(19) Europäisches Patentamt / European Patent Office / Office européen des brevets

(11) **EP 3 017 762 A1**

(12) **EUROPEAN PATENT APPLICATION**

(43) Date of publication:
**11.05.2016 Bulletin 2016/19**

(51) Int Cl.:
***A61B 5/103*** *(2006.01)*     ***A61B 5/024*** *(2006.01)*
***A61B 5/113*** *(2006.01)*

(21) Application number: **15193735.6**

(22) Date of filing: **03.02.2012**

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br><br>(30) Priority: **08.02.2011 US 201161462811 P**<br><br>(62) Document number(s) of the earlier application(s) in<br>accordance with Art. 76 EPC:<br>**12745140.9 / 2 672 891**<br><br>(71) Applicant: **Board of Trustees of the University<br>of Arkansas<br>Little Rock, AR 72207-3608 (US)** | (72) Inventors:<br>• **Eswaran, Hari<br>Little Rock, AR 72212 (US)**<br>• **Lowery, Curtis L.<br>Little Rock, AR 72223 (US)**<br>• **Wilson, James D.<br>Bendon, AR 72015 (US)**<br><br>(74) Representative: **Williams Powell<br>11 Staple Inn<br>London WC1V 7QH (GB)**<br><br>Remarks:<br>This application was filed on 09.11.2015 as a<br>divisional application to the application mentioned<br>under INID code 62. |

(54) **PNEUMATIC TOCODYNAMOMETER**

(57) A pneumatic tocodynamomeer ("pTOCO") (10) having a guard-ring (13) with a thin elastic membrane (18) stretched across a shallow spherical depression (12) trapping a small volume of air in the centre of the guard-ring (13). A pressure transducer (17) may be moulded into the body (11) of the pTOCO (10). In an alternative embodiment, the air volume beneath the membrane (18) of the pTOCO (10) may be connected via a low volume air conduit (14, 15) to a separate pressure transducer (16).

FIG.12

EP 3 017 762 A1

## Description

The present invention relates to a tocodynamometer, and in particular, to a pneumatic tocodynamometer.

In clinical practice, uterine contractions during labor are measured using an external strain gauge based tocodynamometer (TOCO) and/or an intrauterine pressure catheter (IUPC). Both devices have distinct advantages and disadvantages in assessing frequency, amplitude and duration of contractile activity.

The standard for a TOCO design is the "guard-ring" style developed by Smyth et al. in the 1950s. The guard-ring TOCO comprises a strain gauge supported within a rigid ring. The strain gauge of the guard-ring TOCO has very low compliance; i.e., it does not move when force is applied. The guard-ring TOCO has the disadvantages of relatively high cost and not being disposable, although it is frequently unintentionally disposed of. Disposable TOCO's are desirable to avoid cross contamination between patients.

It is therefore desirable to provide a low cost, disposable alternative to the standard guard-ring TOCO while maintaining operational equivalence to this standard device.

The present invention is directed to a device that satisfies this need. The apparatus is a pneumatic tocodynamometer ("pTOCO") that comprises a guard-ring with a thin elastic membrane stretched across a shallow depression, for example a spherical depression, in the center of the guard-ring. The elastic membrane traps a small volume of air in the depression. A pressure transducer may be molded into the body of the pTOCO. In an alternative embodiment, the air volume beneath the membrane of the pTOCO may be connected via a low volume air conduit to a separate pressure transducer.

In either version, it is a significant aspect of the invention that the operational response of the pTOCO is substantially equivalent to that of the standard guard-ring TOCO. This requires that the pTOCO have essentially the same physical shape, guard-ring area and pressure sensing area. Furthermore, the pressure sensing area presents stiff resistance to applied pressure. In other words, the compliance of the elastic membrane mimics the low compliance of the strain gauge of the guard-ring TOCO. Low compliance implies small displacements of the elastic membrane and small volumes of trapped air volume behind the membrane. A preferred displacement is 0.005 inches (0.0127 cm) during a normal contraction and an acceptable range of volume is from around 1 cc to 5 cc with a most preferred volume of around 1 cc or less. The invention is not limited to these displacement and volumes. Displacements and volumes are acceptable if the operational performance of the device does not substantially depart from the characteristics of the standard guard-ring TOCO.

It is therefore an object of the present invention to provide for a tocodynamometer that is less expensive to manufacture and therefore less costly than the standard strain gauge guard-ring tocodynamometer.

It is a further object of the present invention to provide for a tocodynamometer that is operationally equivalent to the standard guard-ring tocodynamometer.

It is also an object of the present invention to provide for a tocodynamometer that is disposable.

It is a still further object of the present invention to provide for a tocodynamometer that is small and lighter than current tocodynamometers to improve the comfort of the patient.

These and other features, objects and advantages of the present invention will become better understood from a consideration of the following detailed description of the preferred embodiments and appended claims in conjunction with the drawings as described following.

Fig. 1 is a graph showing the response of an embodiment of the present invention of the applied force in grams versus the pressure readout of a fetal heart rate monitor. The initial pressures were made to align at the 5 gram point on the graph. Note that in Fig. 1, the term "air TOCO" is understood to refer to the pTOCO.

Fig. 2 is a graph of pressure applied to a standard TOCO versus the pressure readout on a fetal monitor.

Fig. 3 is a plot of average membrane displacement as a function of initial air volume at a readout of 100 mmHg.

Figs. 4A-D are drawings of a embodiment of a sensor body of the pTOCO of the present invention. Fig. 4A is a top plan cross section view of the sensor body taken along the line A-A of Fig. 4C. Fig. 4B is a detail view of the area within the circle of Fig. 4A. Fig. 4C is a front side elevation view of the sensor body. Fig. 4D is a right side elevation view of the sensor body of Fig. 4C.

Fig. 5 is a plan view of a modified micrometer used to measure the static transfer characteristics of tocodynamometers.

Fig. 6 is a graph of the sets of tocodynamometer responses to membrane displacements as indicated by triangle, circle and cross.

Fig. 7 is a graph of contraction measurements over a time interval of 1000 seconds. The upper trace is the recording from a standard TOCO while the lower trace is the simultaneous recording from the 10 cc/100 cc pTOCO. In the time interval 0 seconds to about 550 seconds, the clamp was closed so that the air volume of the pTOCO was 10cc. After about 550 seconds, the clamp was opened and the air volume of the pTOCO was 100 cc. A dramatic drop in response is observed after the clamp is opened.

Fig. 8 is a perspective view of an example of a prior art tocodynamometer (Type 1).

Fig. 9 is a perspective view of another example of a prior art tocodynamometer (Type 2).

Fig. 10 is a graph of two contractions. The upper trace is the recording from a standard TOCO while the lower trace is the simultaneous recording from the 1 cc pTOCO.

Fig. 11 is a graph of a single contraction. The upper trace is the recording from a standard TOCO while the lower trace is the simultaneous recording from the 10 cc pTOCO.

Fig. 12 is a top plan cross sectional view of an embodiment of a pneumatic tocodynamometer of the present invention along with a block diagram showing connections to a pneumatic pressure transducer and fetal monitor.

Fig. 13 is top plan cross sectional view of an alternative embodiment of a pneumatic tocodynamometer of the present invention having a pneumatic pressure transducer embedded in the sensor body along with a block diagram showing connections to a fetal monitor.

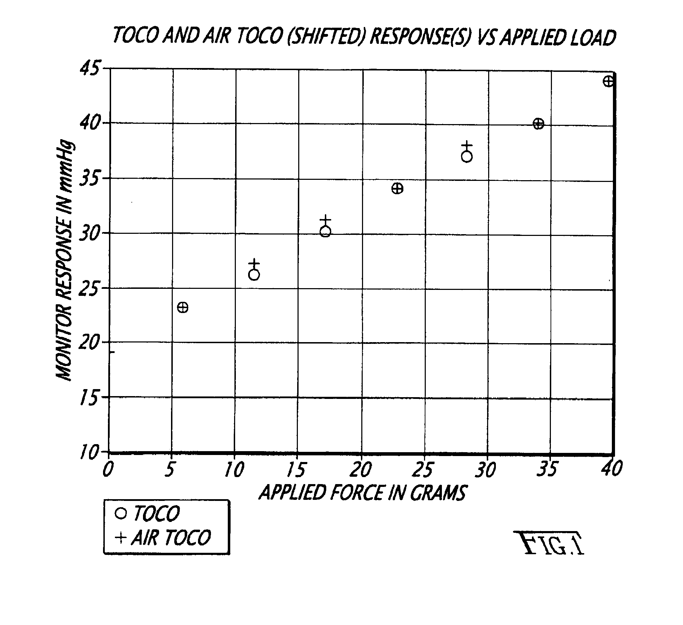

With reference to Figs. 1-13, presently preferred embodiments of the invention may be described.

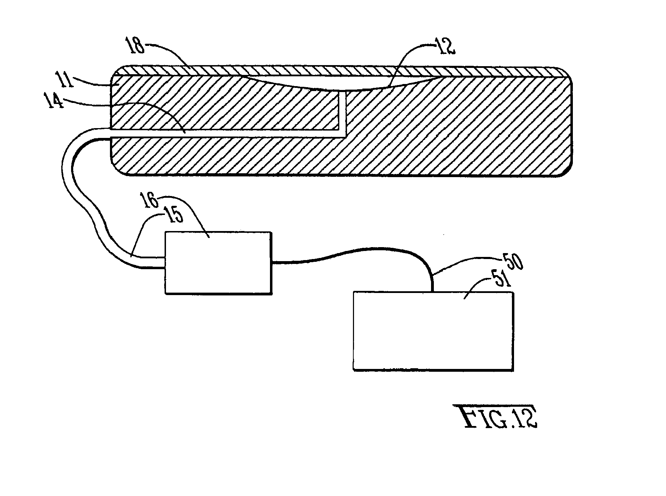

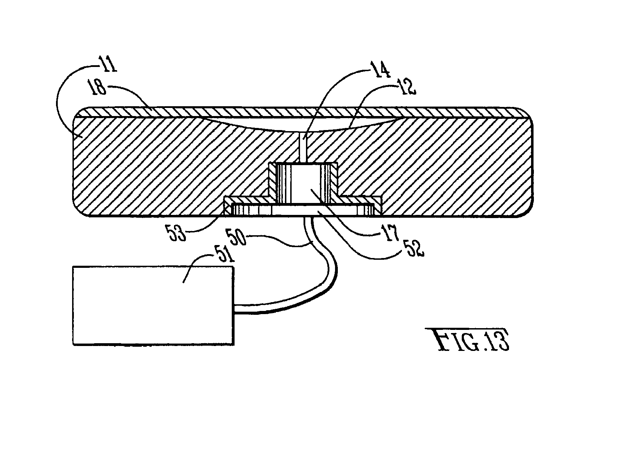

With reference to Figs. 12 and 13, embodiments of the pTOCO 10 include a sensor body 11 provided with a low profile having a shallow depression 12 in the center surrounded by a guard-ring 13. The shallow depression 12 communicates either with a low volume airway 14 to a low volume tube 15 connected to a pressure transducer 16 or directly to a pressure transducer 17 embedded in the sensor body 11. The pressure transducer 17 may be mounted to an electronics board 52. The pressure transducer 17 and electronics board 52 may be placed into a recess 53 in the sensor body 11 so that the pressure transducer 17 is in fluid communication with the airway 14 and thence to the air volume in the depression 12. The electronics board is desirably sealed into the recess 53.

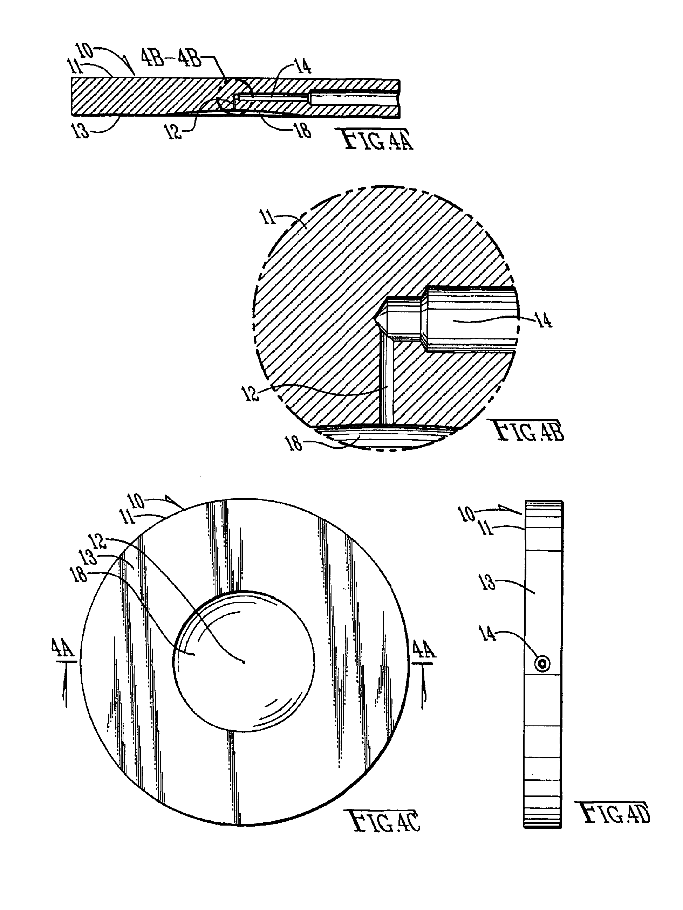

A flexible membrane 18 is stretched across the sensor body 11 of the pTOCO 10 trapping air in a very small volume. The membrane 18 may be attached to the sensor body 11 by adhesive. See Figs. 4A-D and 12 for drawings illustrating one embodiment of the sensor body 10. The sensor body 10 may be made of plastic, such as acetal plastic. The trapped air within the depression 12 plus the air in the airway 14 and air in the tubing 15 collectively comprise a closed interior air volume. Pressure applied to the membrane 18 compresses the trapped air which increases the internal air pressure.

The pressure and volume characteristics are described by the ideal gas law,

$$PV = nRT, \hspace{8cm} Eq.\ (1)$$

where P is pressure, V is volume, n is the number of moles of the gas, R is a constant, and T is the temperature. Assuming that the temperature remains constant and that there is no gas leak, the right hand side of Eq. (1) is a constant. At the pressures measured during contractions, the flexible membrane 18 moves only slightly if and only if the volume, V, is kept small. Said another way, keeping the volume small allows pressure on the membrane 18 to be transmitted to the pressure transducer 16, 17 with almost no membrane movement. The membrane 18 therefore exhibits very low mechanical compliance similar to a standard TOCO 20 and presents the same geometry to the patient. These are essential characteristics required to duplicate the performance of the standard guard-ring TOCO 20.

In the embodiment shown in Fig. 13, a pressure transducer 17 would be molded into the plastic sensor body 11. An electrical connector 50 to a standard fetal monitor 51 would be provided. This embodiment would be completely disposable.

The embodiment of Fig. 12 would have the air volume in the depression 12 connected to a physically separate pressure transducer 16 by a very low volume flexible plastic tube 15. The transducer 16 is in turn connected by an electrical connector 50 to the fetal monitor 51. The plastic parts would be replaceable, allowing the pressure transducer 16 to be reused, but easily replaced. This would offer a very low cost alternative for a disposable TOCO.

A slightly modified device with extended bandwidth could serve as a maternal breathing and pulse monitor during labor. Such maternal parameters collected simultaneously with contractile events could be of great value during labor.

A prototype of the second embodiment described above used an inter-uterine pressure catheter (IUPC) transducer, manufactured by Utah Medical Products, which was connected by a 15 cm length, 1 mm diameter plastic tube 15 to the sensor body 11 shown in Figs. 4A-D. A thin elastic membrane 18 was glued to the surface shown from the side in Fig. 4A (Section A-A), which covered a shallow spherical depression 12 machined into the lower surface, i.e., the surface facing the patient. A desirable size of the depression 12 is a spherical radius of 10 cm and a diameter of 3 cm. The membrane 18 is placed against the patient and held in place by a retaining belt. As pressure is developed, the pressure is coupled to the air trapped inside the small closed interior air volume.

A sensor body 11 similar to that shown in Figs. 4A-D may be manufactured by injection molding, with or without a pressure transducer 16, 17 imbedded in the plastic. Current IUPC manufacturing technology imbeds a disposable pressure transducer in a plastic housing at moderate cost. In the second embodiment with external pressure transducer 16, connecting tubing 15 would be attached to a pressure transducer 16 and fetal monitor 51 as depicted in Figs. 4A-D and 12.

It is anticipated that the pTOCO 10 may be marketed in at least the two embodiments described above: 1) disposable body 11 with embedded pressure transducer 17 and 2) disposable body 11 that is attached to reusable pressure transducer 16. In the first case, the completely disposable device would have an electrical connector cable 50 which plugs into a standard IUPC connector on fetal monitor 51. In the second case, the semi-permanent pressure transducer/amplifier 16 would plug onto the end of the standard IUPC connector and cable. The semi-permanent transducer 16 would be viewed as a one or two inch (2.54 or 5.08 cm) extension of the basic IUPC cable. The semi-permanent pressure transducer 16 could be replaced at modest cost and could be easily removed for IUPC use. The mechanical problems would be making sure that the tube does not get pinched off by the patient and that there is always an air tight connection to the pressure transducer 16. The first embodiment would be the simplest and most reliable, while the second embodiment would be the most cost effective because the pressure transducer would be reusable.

Characteristics of the Standard Guard-Ring TOCO

The present invention is intended to provide a low cost disposable alternative to standard guard-ring TOCO's while maintaining operational equivalence to the standard device. Several measurements and calculations were carried out to better understand the mechanical characteristics of the standard TOCO and the pTOCO. Of principle concern is the static transfer properties of the standard mechanical TOCO compared to the static transfer properties of the pTOCO.

The de facto standard tocodynamometer (TOCO) design is the "guard ring" style. This device was first described in detail in C.N. Smyth, "The Guard-Ring Tocodynamometer," Journal of Obstetrics and Gyneacology, Vol. 64, Issue 1, pp. 59-66, February 1957. The general TOCO dimensions in Smyth's paper indicate an outside diameter of 7.5 cm, a pressure sensitive piston diameter of 2.25 cm, which is very similar to modern versions of the instrument, which may vary from 7.7 cm to 6.5 cm in diameter.

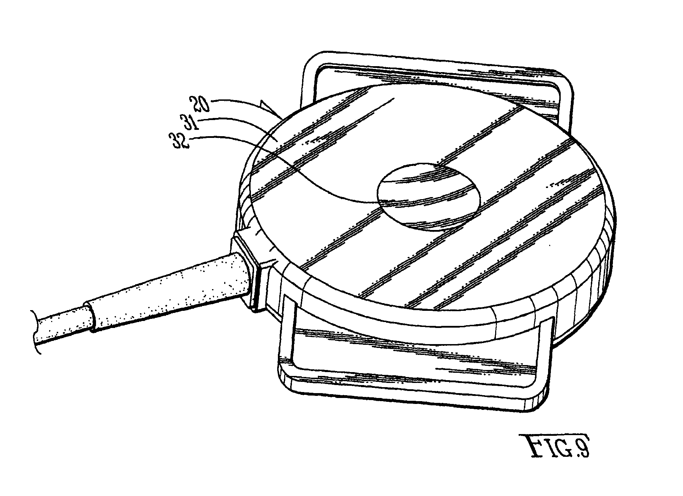

From Figure 9 and the discussion that follows in Smyth's paper, it is clear that the guard-ring tocodynamometer must present a flat surface to the patient's abdomen and that "in practice a flatness +/- 0.02 cm has been found both practicable and sufficient." As used herein, a surface is considered essentially flat or coplanar if it meets this criterion. Smyth also states that "the pressure plate deflection is 0.025 cm for an intra-amniotic pressure of 120 grams/cm$^2$." Although there are variations in the shape of the pressure sensitive piston, current commercial instruments still retain essentially the same mechanical compliance with respect to the pressure sensitive piston.

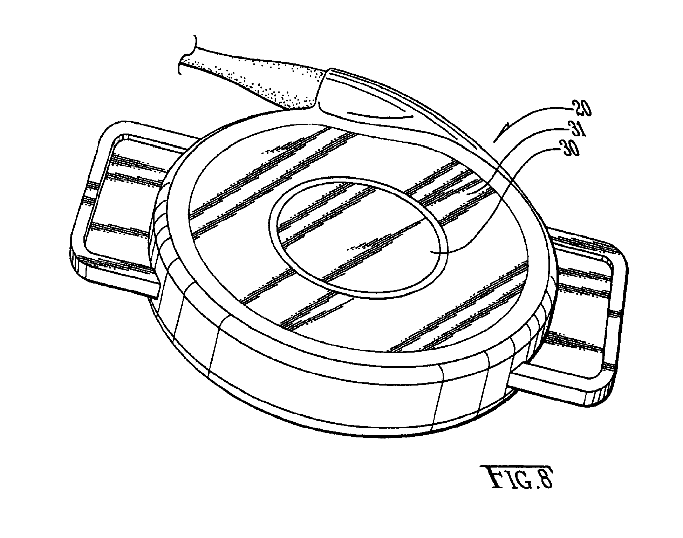

The modern type of TOCO 20 is about 7 cm in diameter with a 3 cm diameter strain gauge force sensor 30 at the center of a guard ring 31. Examples of prior art TOCOs are shown in Figs. 8 and 9. The characteristics are shown in Table 1.

| Table 1 | Type 1 | Type 2 |
|---|---|---|
| Body Diameter | 6.8 cm | 7.7 cm |
| Sensor Diameter | 3.0 cm | 2.5 cm |
| Body Thickness (Center) | 1.8 cm | 1.8 cm |
| Description of Sensor | Flat and hard | Slightly dimpled, but stiff |

The Type 1 force sensor 30 is flat and hard, while the Type 2 force sensor 32 is slightly dimpled. The force sensor 30, 32 has very low compliance (i.e., does not move significantly when force is applied).

It is desirable that the pneumatic TOCO (pTOCO) 10 of the present invention respond in an equivalent fashion to the response of the guard-ring TOCO 20 and that it does not suffer from interferences such as maternal breathing and movement artifacts. As shown below, a pTOCO having a uniformly elastic membrane has acceptable performance up to about 10 cc of closed interior air volume, while a pTOCO having a membrane that is more rigid in the center and therefore responds in a piston-like fashion has an acceptable performance up to about 20 cc closed interior air volume. Above these volumes, interferences such as maternal breathing and movement artifacts degrade performance beyond acceptable levels, while lower air volumes tend to have better performance. While a volume below 1cc would be desirable, 1 cc may be the lowest practically achievable for commercial purposes.

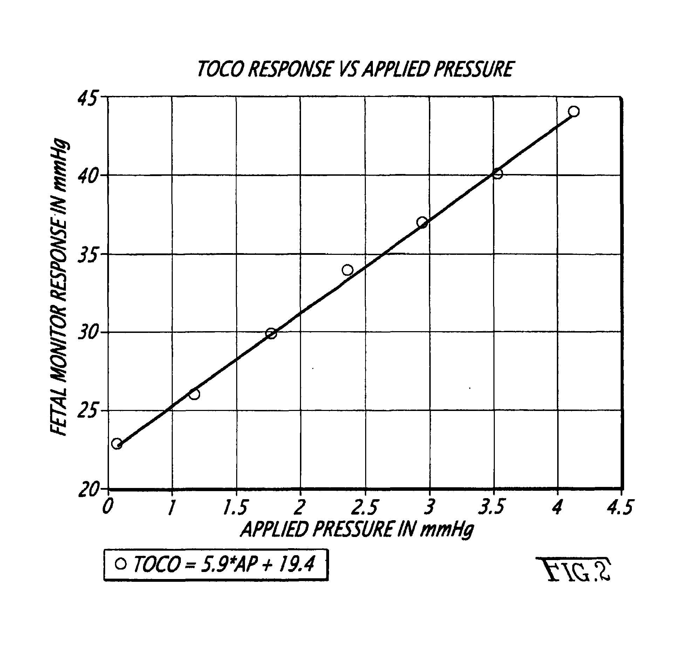

The response of a standard TOCO 20 to pressure applied to the pressure sensing area is approximately in a 6:1 ratio compared to the pressure indicated on a fetal monitor. Note that in Fig. 2 the constant (or "baseline") is not important, because it is removed by a procedure called "setting the baseline" when a TOCO is applied to a patient. This is done once, between contraction events, and is a part of the normal operation of a TOCO. In Fig. 2, pressure was applied to a standard TOCO 20 while reading the pressure indicated on a fetal monitor. Therefore, the pressure transducer 16, 17 used in the pTOCO 10 must be either six times more responsive than a standard IUPC transducer or if a standard IUPC transducer is used, then it will require post amplification. The prototype of the second embodiment used a standard IUPC transducer and consequently was fitted with post amplification electronics to achieve equivalence to a TOCO 20.

The displacement response of the standard TOCO 20 was determined by applying a specially fitted micrometer movement 40. The rigid pressure plate of the standard TOCO must move 9.9 mils (0.0251 cm) to produce a readout of 100 mmHg on the fetal monitor. This is approximately twice the thickness of a piece of paper. A pressure of 100 mmHg is full scale on a fetal monitor strip chart. Typical contractions would be expected to reach approximately half that amplitude, thus a nominal displacement is about 5 mils (0.005 inches) (0.0127 cm).

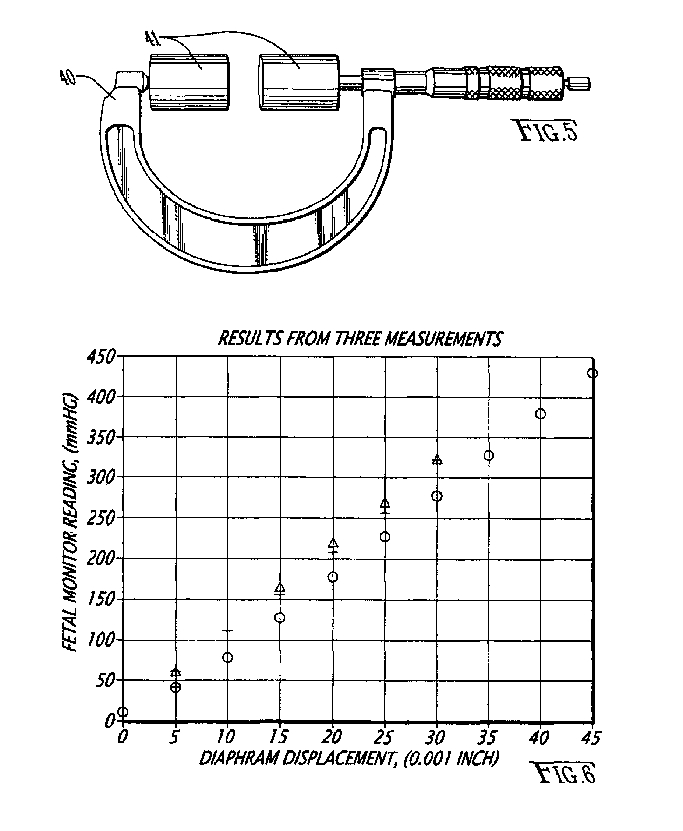

A tool 40 to accurately control the membrane displacement of the TOCO is shown in Fig 5. The device is essentially a standard micrometer 40 modified with 1 inch (2.54 cm) diameter contact surfaces 41 (jaws). The contact surface 41 on the right has an internal steel ball bearing for smooth accurate moments. The micrometer 40 has a 1 mil (1 mil = 0.001 inch = 0.0254 mm) resolution and one complete revolution of the dial gives 25 mil (0.635 mm) displacement. The tool 40 was placed across a standard TOCO and the jaws 41 were closed until contact was made. After contact, the baseline reference button was pressed to make the fetal heart rate monitor read 10 mmHg pressure. After setting the baseline, the tool 40 was adjusted inwardly at regular increments. Fig. 6 presents three sets of measurements on a single standard TOCO.

Fig. 6 shows that the TOCO is in general linear with sensor displacement, but the baseline setting is not consistent. There is some difference in linearity at low displacements. It is noted that non-linearity has been demonstrated with dead weight measurements as well. Since the baseline setting is arbitrary, the only relevant parameter is the slope, which was computed to be 10.1 mmHg/mil ($3.98 * 10^3$ mmHg/cm) using the Matlab® least squares fitting function. Note that a typical piece of paper is about 4 mils thick. A 4 mil displacement would cause a chart deflection of 50.5 mmHg which is typical for the peak pressure reading during a normal contraction using a standard TOCO.

To quantify the responsivity of the TOCO with respect to pressure, thin disc shaped weights were placed on the force sensing area of a standard TOCO 20 and the corresponding pressure readings indicated on the fetal monitor were recorded. The resulting curve was shifted up or down as needed so that at approximately 5 grams the output would be 10 mmHg. This is equivalent to setting the baseline at 5 grams. The process was repeated using a pTOCO prototype. The results are presented in Fig. 1.

Since the TOCO measures the force applied to a stiff pressure plate, the average surface pressure is simply the applied weight divided by the area of the pressure plate. From the dead weight experiments, it is clear that the TOCO indicates a pressure that is almost six times the actual applied pressure. A linear regression gives $P_{readout} = 5.9*P_{applied}$ + constant. Since the pressure developed on the pressure plate of a TOCO outside the body must always be less than the pressure inside the uterus, the abdominal pressure measured by a TOCO is simply scaled by about six to give a value that is comparable to an expected inter-uterine pressure catheter IUPC measurement during labor.

The present invention is intended to reproduce the operational characteristic of a commercial guard ring TOCO 20 and where possible provide for improvement. The relevant characteristics of a standard TOCO 20 are as follows:

    1. Body diameter ~7 cm.
    2. Body thickness - 0.7 cm to 1 cm.
    3. Area of pressure sensitive transducer ~7 cm². If circular, 3 cm diameter.
    4. Displacement of transducer pressure plate of 9.9 mils (0.0251 cm) results in a pressure increase of 100 mmHg on the fetal monitor.
    5. Responsivity is 1/6 (mmHg in versus mmHg out). From dead weight experiments performed earlier, the fetal monitor indicates 100 mmHg pressure when one applies (100/6) mmHg pressure on the pressure plate.

The commercial TOCO 20 shows some nonlinearity at low pressures, but this is not considered important or desirable. The nonlinearity of the standard TOCO 20 at low pressures is thought to be due to slack in the mechanical linkage between the contact surface and the strain gauge. These measurements are taken as representative of all commercial TOCOs but may vary from model to model. Given any one model, the methods presented here show how to design a comparable pTOCO.

The experimental results presented herein illustrate the operational characteristics of particular transducers. While other transducers may show slightly different characteristics, it is believed that the conclusions reached herein are representative of all transducers used in prior art tocodynamometers.

Equivalence to Standard TOCO

For a pTOCO to achieve equivalence to a standard TOCO 20, the pTOCO must have essentially the same physical shape, guard ring area, displacement and pressure sensing active area. A key point is that the pressure sensing area of a TOCO presents stiff resistance to applied pressure.

Now consider the shape an elastic sheet (membrane) would take if stretched across a circular open space of 3 cm diameter and then pressed against the abdomen of a patient in labor. If the membrane has sufficient back side air pressure, then it will remain relatively flat achieving stiff resistance to applied pressure. This is the normal operating condition desired for the pTOCO. If the back side is simply open to room air pressure, then the patient's skin will deform and extend into the open space. The extent of deformation will depend upon the properties of the elastic sheet and the maternal skin so that the exact volume displacement of the membrane 18 can only be characterized experimentally. Assume a 3 cm diameter piston shaped intrusion and assume that the membrane offers no resistance to movement. The largest practical readout during labor is about 100 mmHg. Given the 6:1 reduction in pressure at the abdomen, it is possible to compute the volumetric displacement of the membrane during a 100 mmHg event using Eq. (1). From Eq. (1),

$$P_1V_1 = P_2V_2, \qquad\qquad\qquad \text{Eq. (2)}$$

where the subscripts represent the conditions before "1" and during "2" contractions. The initial pressure, $P_1$ = 760 mmHg, is assumed to be atmospheric and $V_1$ is the assumed volume. $P_2 = P_1 + 100$ mmHg / 6. The volumetric displacement of the membrane, $V_d = V_1 - V_2$. Making substitutions gives:

$$V_d = V_1(1 - P_1/P_2) \qquad\qquad\qquad \text{Eq. (3)}$$

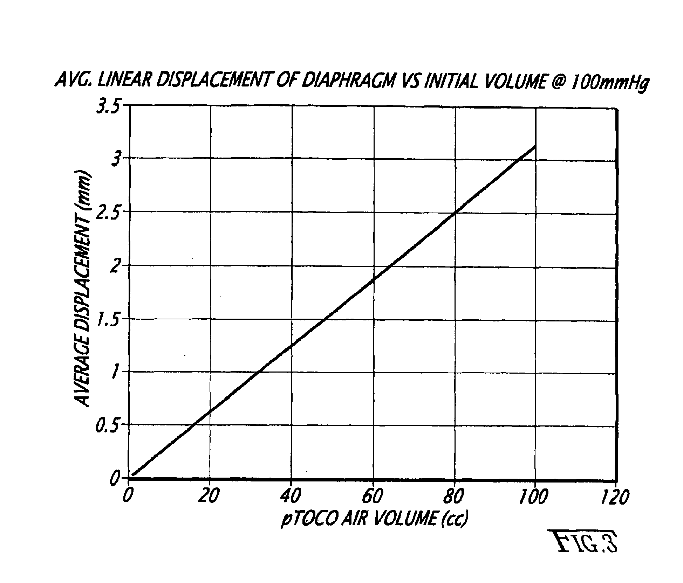

The average linear displacement of the membrane is then found by dividing by the circular area of the membrane, A. See Fig. 3 for a graph of $V_d/A$ versus initial volume.

Fig. 3 demonstrates that very small deformations in the membrane occur at low initial air volumes. For the second prototype, $V_1$ = 0.83 cc (cubic centimeter) resulting in an average membrane displacement of 0.026 mm. If the initial volume is on the order of 1 cc (preferred embodiment), then the membrane and maternal skin will undergo very little stretching, making the measurement independent of the elastic properties of device and maternal tissue. As the trapped air volume increases, the properties of the membrane and maternal tissue become more and more important.

pTOCO Design Considerations

The mechanical action of the TOCO membrane is essentially the same as a spring loaded with a fixed weight which is described by Hooke's law, F = -k*x. The force is produced by pressure developed inside the uterus causing it to rock forward during a contraction which in turn develops a force on the TOCO pressure plate. One can then model output of the instrument with a linear equation:

$$Pm = k*x + C1, \text{ where} \qquad\qquad\qquad \text{Eq. (4)}$$

Pm     is the pressure reading on the fetal monitor in mmHg,
k     is the "displacement constant" of the TOCO pressure plate, and
C1     is a constant set during the baseline zeroing procedure.

From measurements described above, k = $3.98*10^3$ mmHg/cm. In the Smyth paper, an intrauterine pressure of 120 g/cm$^2$ gave a displacement of 0.025 cm. Given that the density of Hg is 13.534 g/cm$^3$, the 'k' for the Smyth instrument is $3.5466*10^3$ mmHg/cm. Further the pressure actually displayed on the monitor (Pm) as defined above is proportional to the actual pressure (P) applied to the' TOCO pressure plate such that

$$Pm = G*P + C2 \qquad\qquad\qquad \text{Eq. (5)}$$

From measurements made above, G = 5.9. As before, C2 is an arbitrary constant set by the baseline zeroing procedure. If the analysis is limited to changes in pressure, $\Delta Pm$ and $\Delta P$, then C1 and C2 can be ignored. It is noted that Smyth did not calibrate his instrument to read out equivalent intrauterine pressure, so we do not have a 'G' value from Smyth's work. However, Smyth indicates that a 100 g weight placed on the 5 cm$^2$ pressure plate, a pressure of 20 (g/cm$^2$) = 14.77 mmHg, would produce a deflection of 10 cm on 11 inch chart paper - just less than half of full scale. In our case, 14.77 mmHg would be 87% of full scale. Therefore, the value of 'G' that Smyth used must have been comparable. The value of 'G' seems to be somewhat arbitrary in that we must choose a scale factor that gives a $\Delta Pm \sim 50$ mmHg for a "typical" contraction which is somewhat subjective.

An amplifier will typically be required between the pneumatic tocodynamometer and the IUPC. In order to achieve operational equivalence with prior art tocodynamometers, the gain of the amplifier may be adjusted as necessary. The gains suggested herein are believed to be typical, but particular circumstances may require the gain of the amplifier to be set at different levels.

The pTOCO design must satisfy both Eq. (4) and Eq. (5) to duplicate the exact behavior of a standard TOCO. To satisfy Eq. (5), the pressure transducer amplifier is set to readout 5.9 times the applied pressure, P. Before attempting to match the performances of the pTOCO and TOCO, we need to derive basic equations that describe the behavior and to develop some critical ideas. Starting from Boyle's law, $P_1V_1 = P_2V_2$, it is easy to show that

$$\Delta P = P_2 - P_1 = P_1(V_1 - V_2)/V_2 \qquad \text{Eq. (6)}$$

If we now let $V_2 = V_1 - \Delta V$, where $\Delta V$ is the small decrease in volume (membrane deformation) caused by the applied pressure change $\Delta P$, we find that

$$\Delta P = P_1{}^*\Delta V/(V_1 - \Delta V) \qquad \text{Eq. (7)}$$

Eq. (7) can be simplified if $V_1 >> \Delta V$. Therefore we solve Eq. (7) for $\Delta V$ giving

$$\Delta V = (\Delta P/(P_1 - \Delta P))\, V_1 \qquad \text{Eq. (8)}$$

$P_1$ is taken to be atmospheric pressure, 760 mmHg. $\Delta P$ can be estimated by making the following assumptions: the normal base line procedure is followed and the monitor output is set to 10 mmHg at baseline. Then sufficient pressure is applied to the pTOCO to force a read out of 60 mmHg on the monitor strip chart, which is taken as a typical contraction. That means that $\Delta Pm$ is 50mmHg. From Eq. (5), it follows that $\Delta P = 50/5.9$ mmHg = 8.47 mmHg. For typical conditions, $\Delta V = (0.0112)\, V_1$ showing that $\Delta V$ is only about 1 % of the trapped air volume, $V_1$. To a good approximation, Eq. (8) simplifies to

$$\Delta P = P_1{}^*\Delta V/V_1 \qquad \text{Eq. (9)}$$

Clearly the change in volume, $\Delta V$, is related to the amount of movement in the membrane. What is needed then is a model that relates $\Delta V$ to $\Delta x$. Two models are analyzed, a piston model and a spherical model.

Some commercial tocodynamometers are similar to the guarding tocodynamometer described by Smyth, but in these devices the pressure sensitive plate has a raised conical surface that pushes into the maternal skin by a few millimeters. The pTOCO can be adapted by gluing a conical disc to the surface of the pTOCO membrane. Optionally, the center of the pTOCO membrane can be made rigid by gluing a disc to the inside of the membrane. In either case, the modified pTOCO membrane would act like a piston - free to move at the edges, but rigid in the center. For a piston design, we can say that $\Delta V = A^*\Delta x$ where A is the area of the rigid pTOCO membrane. Using the various equations and equating constants gives equations that describe the 'k' of the piston model.

$$k = P_1{}^*A^*G/V_1 = P_1{}^*G/X_m \qquad \text{Eq. (10a)}$$

$$V_1 = P_1{}^*A^*G/k \qquad \text{Eq. (10b)}$$

Letting $P_1 = 760$ mmHg, A = 7.07 $cm^2$ (3 cm diameter), G = 5.9, and k = $3.98^*10^3$ mmHg/cm and solving for $V_1$ gives $V_1 = 7.96$ cc for the piston type membrane and the given geometry. It can be seen in Eq. (10b), that the required volume, $V_1$, is proportional to the area, A, and is therefore dependent upon the selected geometry. Equation (10a) includes the term $X_m$, where $X_m = (V_1/A)$ is the mean depth of the trapped air volume behind the unloaded pTOCO membrane. For the stated values, $X_m = 1.126$ cm.

The above values for $X_m$ and $V_1$ are maximum values for acceptable performance for the stated case. Smyth's criteria is that the membrane displacement be limited ($\Delta x <= 0.025$ cm), which is accomplished by setting the value of 'k' for the expected pressures. However, there is no lower bound on the displacement, $\Delta x$. Making $X_m$ and $V_1$ small

increases 'k' and therefore minimizes the displacement $\Delta x$.

The effects of the elastic properties of the membrane can now be analyzed. The membrane requires force to displace and as such has a 'k' value. For a pTOCO the 'k' for the membrane simply adds to the 'k' due to $\Delta P$. The equation that describes the system is

$$k_{pTOCO} = k_{membrane} + k_{air} \quad \text{where,} \qquad \text{Eq. (11)}$$

$k_{pTOCO}$ is the 'k' of Eq. (4),
$k_{membrane}$ is the displacement constant for the membrane, and
$k_{air}$ is the 'k' computed from Eq. (10a).

What we see is that the effect of the membrane is to make the membrane 'stiffer' as compared to the stiffness due to the trapped air acting alone. If $k_{membrane} \sim k_{air}$, the consequence is that for a given applied $\Delta P$ at the membrane the resulting $\Delta x$ is smaller than expected which in turn produces a smaller $\Delta V$ and therefore a smaller than expected $\Delta P$ at the pressure sensor. To compensate for a stiff membrane, one must simply increase the gain, G. It is desirable to maximize $k_{air}$ by making $V_1$ as small as practical. This reduces the effect of the membrane and maximizes the pressure delivered to the pressure sensor. As a design check, if the G needed for the pTOCO is found to be 5.9, then the membrane stiffness must be insignificant with respect to the stiffness presented by the trapped air volume.

Without an exact model of maternal tissue and the application of finite element software, there is no practical way to compute exactly how the pTOCO membrane deforms during a contraction. We employ a spherical model as an approximation. This assumption gives a more realistic model to work with than assuming a rigid membrane. The spherical model gives

$$\Delta V = \pi h^2(3r-h)/3, \text{ where} \qquad \text{Eq. (12)}$$

r is the radius of the sphere, and
h is the displacement of the membrane at its center.

As a further constraint, the edge of the volume $V_d$ must be 3 cm wide. This gives the following constraining equation with units in cm:

$$r^2 = (3/2)^2 + (r - h)^2 \qquad \text{Eq. (13)}$$

It is straightforward to expand the right hand side of Eq. (13) and solve for the radius, 'r'.

$$r = ((3/2)^2 + h^2)/(2h) \qquad \text{Eq. (14)}$$

Then Eq. (14) can be substituted into Eq. (12) to eliminate 'r', giving:

$$\Delta V = \pi h(3(3/2)^2 + h^2)/6 = 3.5343{*}h + 0.5236{*}h^3 \qquad \text{Eq. (15)}$$

If the central depression, h, is limited to a maximum of 0.025 cm, then the $h^3$ term is at least four orders of magnitude smaller than the first term and for a 3 cm wide spherical depression, we have

$$\Delta V = 3.5343{*}h \qquad \text{Eq. (16)}$$

For a displacement, h, at the center of the membrane, the volume, $\Delta V$, is half that of the piston model for the same displacement. Following the same process as for Eq. (8a) and Eq. (8b), the relationship between 'k' and $V_1$ for the spherical model is given by

$$k = 3.5(cm^2){*} \, P_1{*}G/V_1 = 0.5{*}P_1{*}G/X_m \qquad \text{Eq. (17a)}$$

$$V_1 = 3.5(cm^2)* P_1 * G/k \qquad \qquad Eq. (17b)$$

As before, letting $P_1$ = 760 mmHg, 3 cm diameter membrane spherical model, G = 5.9, and k = $3.98*10^3$ mmHg/cm and solving for $V_1$ gives $V_1$ = 3.98 $cm^3$ for the spherical model and the given geometry. Eq. (17a) includes the term $X_m$, where $X_m = (V_1/A)$ is the mean depth of the trapped air volume behind the unloaded pTOCO membrane. For the stated values, $X_m$ = 0.563 cm.

The minimum practical trapped air volume for the 3 cm diameter membrane is about 1 cc and is the favored implementation. Assume that the trapped volume $V_1$ = 1 $cm^3$ and $\Delta P$ = 8.47 mmHg as before, then $\Delta V$ = 0.0111 $cm^3$. Using Eq. (16), h = 0.003153 cm = 1.242 mils for the given conditions and k = $1.585*10^4$ mmHg/cm which indicates that the pTOCO membrane is about 3.9 times stiffer that the example TOCO.

10 cc and 100 cc Prototypes

A 10 cc and a 100 cc prototype using uniformly elastic membranes were tested on five patients. The 100 cc prototype never produced a usable signal while the 10 cc did. Though not statistically verified, the 10 cc prototype trace appeared to be lower in amplitude and more sensitive to maternal movements such as breathing as compared to 1 cc pTOCO and standard TOCO.

The mean depth behind the membrane, $X_m$, is equal to the total trapped volume (including air in connecting tubing and pressure sensor) divided by membrane area. It was shown that $X_m$ is the primary parameter of determining responsivity and not total volume, $V_1$. The 100 cc prototype TOCO was not functional with $X_m$ = 14.147 cm.

It is understood that the pTOCO membrane cannot have precisely the same characteristics as the stiff plate of the standard TOCO. A realistic spherical model was developed for analysis of the membrane displacement. Assuming a spherical model where the central displacement matches the displacement of the standard TOCO 20, gives $X_m$ on the order of 0.563 cm ($V_1$ = 3.98 cc). This establishes what is likely to be the largest acceptable volume for a pTOCO with 3 cm diameter membrane.

If the membrane is to be made as stiff as possible, then the volume, $V_1$, must be minimized. We have demonstrated a $V_1$ = 1 cc prototype, which gives $X_m$ = 0.1415 cm. The latter case is the most desirable for the following reasons: maximum flatness of the membrane, less membrane tension (less error), and minimum $X_m$ that makes possible a thin tocodynamometer.

Only the 1 cc pTOCO rejected maternal movement artifacts as well as the standard TOCO, while both the 1 cc and 10 cc have similar responsivity. This implies that membrane stiffness is essential for artifact free measurement of maternal contractions. The integration of the concept of a pTOCO with the guard-ring TOCO geometry is essential for FDA (U.S. Food and Drug Administration) equivalency. Achieving artifact free equivalence would limit the $X_m$ to about 0.56 cm or less (~4cc volume). Further, it appears that simply having a small volume pTOCO is not equivalent to small volume plus guard-ring.

As noted above, the response of a standard TOCO 20 to pressure applied to the pressure sensing area is approximately in a 6:1 ratio compared to the pressure indicated on a fetal monitor. Note that in Fig. 2 the constant (or "baseline") is not important, because it is removed by a procedure called "setting the baseline" when a TOCO is applied to a patient. This is done once, between contraction events, and is a part of the normal operation of a TOCO. In Fig. 2, pressure was applied to a standard TOCO 20 while reading the pressure indicated on a fetal monitor. Therefore, the pressure transducer 16, 17 used in the pTOCO 10 must be either six times more responsive than a standard IUPC transducer or if a standard IUPC transducer is used, then it will require post amplification. The prototype of the second embodiment used a standard IUPC transducer and consequently was fitted with post amplification electronics to achieve equivalence to a TOCO 20.

Volume and Displacement in 10 cc and 100 cc Prototypes

To study the effect of volume on the response of the pTOCO, a combination 10 cc or 100 cc volume tocodynamometer was constructed for testing. This prototype is the same diameter as the original 1 cc volume prototype and has the same 3 cm diameter membrane material. The 10 cc prototype was fitted with a large syringe set to 90 cc volume. A "T" connection was placed in the tubing linking the 10 cc pTOCO to the pressure transducer and the syringe was tied into the "T" connection. A clamp was placed between the syringe and the "T" so that if the clamp is closed the volume is - 10 cc and if the clamp is open the volume is - 100 cc. The first case will be referred to as the 10 cc pTOCO and the second case as the 100 cc pTOCO.

From the above measurements, it is clear that the trapped air volume of the pTOCO affects the responsivity of the system for a given membrane displacement. It would be desirable to duplicate the behavior of a commercial tocodynamometer. A 100 mmHg change in pressure should correspond to about 9.9 mil (0.25 mm) change in membrane position. For comparison, a sheet of paper is typically 4 mils (0.1 mm) thick. For an air based pTOCO, the following formula holds:

$$dP = -P_1(dV/V_1)/(1+(dV/V_1)), \qquad\qquad \text{Eq. (18)}$$

where $P_1$ is the original pressure (1 atmosphere or 760 mmHg),
$V_1$ is the original volume,
dV is the change in volume caused by membrane displacement, and
dP is the change in pressure that must be sensed for readout.

The way Eq. (18) is cast makes it clear that the relevant parameter is the percent change in volume ($dV/V_1$). In this case, we do not make the simplification of Eq. (9). If we assume that the pressure sensing area is stiff so that the membrane acts like a piston, as is the case for the commercial TOCO, then we can say something about the equivalent thickness of the trapped air volume. Eq. (18) can then be modified to express dP as a function of displacement. Let $X_m$ = $V_1/A$ so that $V_1 = A*X_m$ and $dV = A*dX$ where A is the area of the membrane ($cm^2$) and $X_m$ is the average depth of the volume behind the membrane. It must be noted that this "average depth" volume would include the volume of tubing used to connect to the pressure transducers and similar volumes not immediately behind the membrane. Eq. (18) reduces to:

$$dP = -P_1(dX/X_m)/(1+(dX/X_m)) \qquad\qquad \text{Eq. (19)}$$

The elastic membrane acts as a piston so that the volume displaced by the membrane can be considered to be a disc shaped volume. As shown above, the displacement of the transducer plate of a standard TOCO is typically 9.9 mills (0.0251 cm) for a 100 mmHg readout. For a 100 mmHg readout, the actual applied pressure is about 1/6 of 100 mmHg which is 16.67 mmHg. Assuming dP = 16.67 mmHg, dX = -0.0251 cm, and $P_1$ = 760 mmHg one gets $X_m$ = 1.17 cm from Eq. (19). Then $X_m$ = 1.17 cm times 7.07 $cm^2$ gives a volume of 8.27 cc.

Let the center of the 3 cm diameter membrane move 9.9 mills (0.0251 cm), then by Eq. (15) we have $V_d$ = 0.0889 $cm^3$. $V_1$ can be found by using Eq. (18) using $dV = -V_d$, dP = 100/6 = 16.67 mmHg, and $P_1$ = 760 mmHg. From Eq. (18), $V_1$ = 4.14 $cm^3$. Also the average thickness would be $X_m$ = 4.14 $cm^3$/7.07 $cm^2$ = 0.563 cm.

If we assume that the membrane is as "stiff" as possible, the volume $V_1$ should simply be minimized. We have demonstrated that a volume of approximately 1 $cm^3$ is possible to achieve. If $V_1$ = 1 $cm^3$, $P_1$ = 760 mmHg, and dP = 16.67 mmHg, then dV = -0.0215 $cm^3$. Assuming a piston movement, the displacement would be dX = 0.003 cm and assuming the more realistic spherical model, h = 0.0061 cm. In either case, $X_m$ = $V_1$/7.07 = 0.1415 cm.

Given the 3 cm diameter membrane used in the prototypes, the area "A" is 0.07 $cm^2$. For volumes of 1 cc, 10 cc, and 100 cc, $X_m$ is 0.145 cm, 1.45 cm, and 14.5 cm respectively. There is a practical limit to the area A, however, the range of volumes that have the same $dX/X_m$ can be quite large.

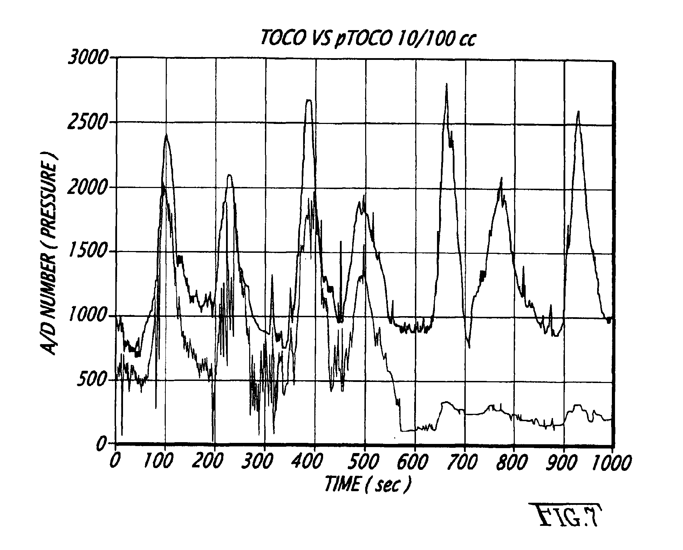

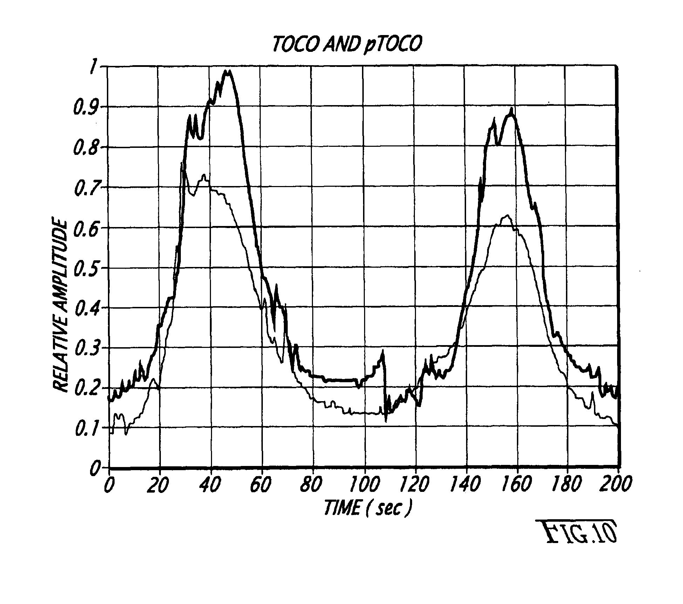

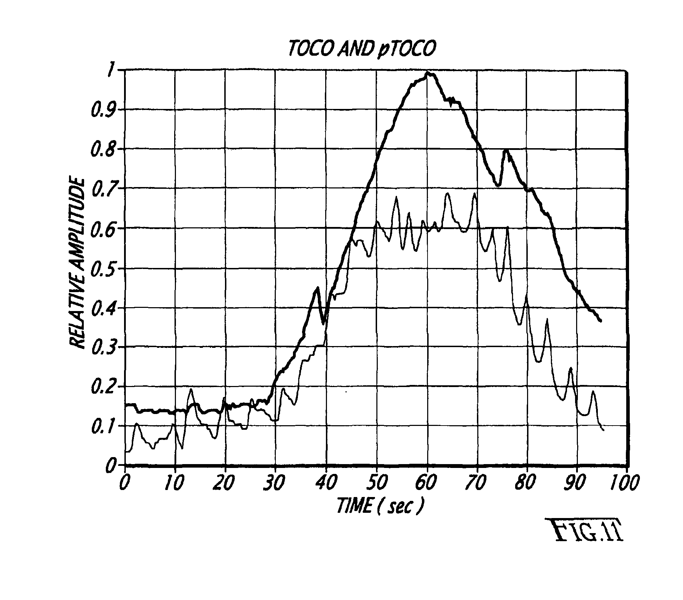

A number of tests were undertaken to evaluate the effectiveness of the 10 cc versus the 100 cc pTOCO keeping the general dimensions of the 1 cc pTOCO (7 cm diameter body and 3 cm diameter membrane). Five sets of data were recorded with the 10 cc volume and 100 cc volume (clamp closed, then open). The protocol called for the pTOCO to be placed on the patient and baseline set in 10 cc mode. After recording, the clamp was opened and the baseline was reset. Recordings were nominally 1200 seconds. The general result was that the 10 cc pTOCO recorded contractions, but displayed 1) much more maternal breathing and 2) more movement artifacts than a standard TOCO or the 1 cc pTOCO. Five recordings did not provide sufficient data for statistical analysis, but the impression is that the magnitude response is reduced somewhat comparing the 1 cc pTOCO to the 10 cc pTOCO. The lack of response from the 100 cc pTOCO was obvious. An example of 10 cc pTOCO versus 100 cc pTOCO is shown in Fig. 7. The clamp was closed (10 cc mode) from time 0 seconds to about 550 seconds. After 550 seconds, the clamp was open and the prototype was operating in the 100 cc mode. The drop in amplitude is obvious at the time the clamp was released. The difference in recorded amplitude between 10 cc and 100 cc pTOCO was not always as large as shown in Fig. 7. However, the 100 cc pTOCO never returned usable recordings. Also, note the large fluctuations (movement artifacts) in the 10 cc pTOCO (lower trace) that are not seen in the TOCO (upper trace). Figs. 10 and 11 show contraction measurements in more detail. Fig. 11 is an expanded view of a single contraction on a scale that makes the graph look more like the strip chart from a fetal monitor. In Fig. 11, the upper trace is the recording from a standard TOCO capturing one contraction simultaneously with a 10 cc pTOCO. The spikes riding on the pTOCO trace are semi-periodic and about 3-5 seconds apart. This is the maternal breathing. Fig. 10 is an example of the 1 cc pTOCO and a standard TOCO capturing data simultaneously (two contractions). Clearly the onset of the pressure increase is the same for both devices and usually the timing of the peak is the same. One can see a sudden change of level at about 110 seconds in the TOCO signal but not at the pTOCO signal. Here the patient was moving and apparently shifted the position of the TOCO but not the pTOCO. Proving equivalence of the two devices has the inherent difficulty that position affects the reading and the patient can move or push on one device and not the other. One must look at the behavior statistically.

There are 4 million births each year in the U.S.A. alone. Adding the industrialized countries where the product would enjoy patent protection would easily double that potential. The second version may be very appealing in cost sensitive situations. All components can be mass produced, including the pressure transducer, at prices comparable to standard disposable IUPC devices.

The present invention has been described with reference to certain preferred and alternative embodiments that are intended to be exemplary only and not limiting to the full scope of the present invention as set forth in the appended claims. The disclosure of U.S. Provisional Application No. 61/462,811 filed 08 February 2011 and of EP 12745140.9, from which the present application is divided, is hereby incorporated herein by reference.

According to another aspect of the present invention there is provided a pneumatic tocodynamometer, comprising: a sensor body having a planar lower surface; said planar lower surface comprising a rigid guard-ring and an elastic membrane covering a depression centered in said rigid guard-ring; a closed interior air volume defined by said depression and said elastic membrane, and a pressure transducer communicating with said closed interior air volume.

The pressure transducer may be embedded within said sensor body.

The pneumatic tocodynamometer may further comprise a connecting tubing communicating with said pressure sensor and an air conduit within said sensor body communicating between said closed interior air volume and said connecting tubing, wherein said closed interior air volume further comprises an air volume contained within said air conduit and said connecting tubing.

The depression may be a spherical depression.

The spherical depression may be defined by a spherical radius of 10 cm and a diameter at said lower surface of 3 cm.

The closed interior air volume may be 1 cc or less.

The closed interior air volume may be in the range of 1 cc to 5 cc.

The elastic membrane may be displaced 0.005 inches (0.0127 cm) or less during a normal contraction.

The pneumatic tocodynamometer may further comprise a fetal monitor operatively connected to said pressure transducer.

According to another aspect of the present invention, there is provided a pneumatic tocodynamometer, comprising: a sensor body; a rigid ring disposed on said sensor body, said rigid ring having a planar lower surface; a depression in said sensor body and centered in said rigid ring; an elastic membrane covering said depression and disposed in a plane that is essentially coplanar with said lower surface of said rigid ring; a closed interior air volume defined by said depression and said elastic membrane; and a pressure transducer communicating with said closed interior air volume.

The membrane may cover substantially the entirety of said lower surface of said sensor body.

The membrane may be sealed to an edge of said depression.

The pneumatic tocodynamometer may further comprise a connecting tubing communicating with said pressure sensor and an air conduit within said sensor body communicating between said closed interior air volume and said connecting tubing, wherein said closed interior air volume further comprises an air volume contained within said air conduit and said connecting tubing.

The membrane may be uniformly elastic.

The membrane may comprise a rigid centre portion and an elastic periphery.

The rigid centre portion may comprise a rigid disc affixed to an interior side of said membrane.

The rigid centre portion may comprise a conical disc affixed to an exterior side of said membrane.

The closed interior air volume may be 10 cc or less.

The closed interior air volume may be around 4 cc.

The closed interior air volume may be around 20 cc or less.

The closed interior air volume may be around 8 cc.

The sensor body may comprise a circular lower surface having a diameter in the range of around 7.7 cm to 6.5 cm.

The mean depth of trapped air behind said membrane may be 1.17 cm or less.

**Claims**

**1.** A pneumatic tocodynamometer, comprising:

a sensor body comprising a rigid ring, said rigid ring having a planar lower surface;

a depression centred in said rigid ring;

an elastic membrane covering said depression and disposed in a plane that is essentially coplanar with said lower surface of said rigid ring;

a closed interior air volume defined by said depression and said elastic membrane; and

a pressure transducer communicating with said closed interior air volume.

2. A pneumatic tocodynamometer as claimed in claim 1, wherein said membrane covers substantially the entirety of said lower surface of said sensor body.

3. A pneumatic tocodynamometer as claimed in claim 1, wherein said membrane is sealed to an edge of said depression.

4. A pneumatic tocodynamometer as claimed in claim 1, further comprising a connecting tubing communicating with said pressure sensor, an air conduit within said sensor body communicating between said closed interior air volume and said connecting tubing, wherein said closed interior air volume further comprises an air volume contained within said air conduit and said connecting tubing.

5. A pneumatic tocodynamometer as claimed in claim 1 or 4, wherein said membrane is uniformly elastic.

6. A pneumatic tocodynamometer as claimed in claim 1 or 4, wherein said membrane comprises a rigid centre portion and an elastic periphery.

7. A pneumatic tocodynamometer as claimed in claim 6, wherein said rigid centre portion comprises a rigid disc affixed to an interior side of said membrane.

8. A pneumatic tocodynamometer as claimed in claim 6, wherein said rigid centre portion comprises a conical disc affixed to an exterior side of said membrane.

9. A pneumatic tocodynamometer as claimed in claim 1 or 4, wherein said sensor body comprises a circular lower surface having a diameter in the range of around 7.7 cm to 6.5 cm.

10. A pneumatic tocodynamometer as claimed in claim 9, wherein a mean depth of trapped air behind said membrane is 1.17 cm or less.

11. A pneumatic tocodynamometer as claimed in claim 6, wherein said closed interior air volume is 20 cc or less.

12. A pneumatic tocodynamometer as claimed in 5, wherein said closed interior air volume is 10 cc or less.

13. A pneumatic tocodynamometer as claimed in claim 5, wherein said closed interior air volume is 4 cc or less.

TOCO AND AIR TOCO (SHIFTED) RESPONSE(S) VS APPLIED LOAD

FIG.1

## TOCO RESPONSE VS APPLIED PRESSURE

○ TOCO = 5.9*AP + 19.4

FIG.2

AVG. LINEAR DISPLACEMENT OF DIAPHRAGM VS INITIAL VOLUME @ 100mmHg

FIG.3

10

11

4B—4B

14

13

12

18

FIG.4A

11

14

12

18

FIG.4B

10

11

12

13

18

4A

4A

FIG.4C

10

11

13

14

FIG.4D

FIG.5

RESULTS FROM THREE MEASUREMENTS

FETAL MONITOR READING, (mmHG) vs DIAPHRAM DISPLACEMENT, (0.001 INCH)

FIG.6

FIG. 7

FIG.8

FIG.9

TOCO AND pTOCO

FIG.10

TOCO AND pTOCO

FIG.11

FIG.12

FIG.13

Europäisches
Patentamt

European
Patent Office

Office européen
des brevets

# EUROPEAN SEARCH REPORT

Application Number

EP 15 19 3735

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 324 259 A (WRIGHT BASIL M) 13 April 1982 (1982-04-13) | 1-5,9, 10,12,13 | INV. A61B5/103 |
| Y | * column 1, lines 36-50, 67 * * column 1, line 67 - column 2, line 13 * * column 2, lines 14-37 * * figure 1 * | 6-8,11 | A61B5/024 ADD. A61B5/113 |
| X | WO 99/19704 A1 (NOVATRIX INC [US]) 22 April 1999 (1999-04-22) * page 6, line 13 * * page 7, line 34 - page 8, line 3 * * page 9, lines 21-23, 30 * * page 10, lines 9-20 * * page 11, line 1 * * page 14, line 18 * * figures 11,13 * | 1,5,9, 10,12,13 | |
| Y | C N Smyth ET AL: "The guard-ring tocodynamometer; absolute measurement of intra-amniotic pressure by a new instrument", The Journal of obstetrics and gynaecology of the British Empire, 1 February 1957 (1957-02-01), page 59, XP055134523, Retrieved from the Internet: URL:http://www.ncbi.nlm.nih.gov/pubmed/134 06636 [retrieved on 2014-08-13] * the whole document * * especially page 64, second column, lines 27-37 as well as figures 8 and 9 * | 6-8,11 | |
| A | US 5 195 536 A (SILVA FIDEL H [US] ET AL) 23 March 1993 (1993-03-23) * column 1, lines 14-26 * * column 2, lines 40-41 * * column 4, lines 46-50 * | 1-13 | |

TECHNICAL FIELDS SEARCHED (IPC)

A61B

-/--

The present search report has been drawn up for all claims

| Place of search | Date of completion of the search | Examiner |
|---|---|---|
| The Hague | 1 April 2016 | Trattner, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 15 19 3735

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 4 559 953 A (WRIGHT BASIL M [GB] ET AL) 24 December 1985 (1985-12-24) * the whole document * ----- | 1-13 | |

TECHNICAL FIELDS
SEARCHED (IPC)

The present search report has been drawn up for all claims

| Place of search | Date of completion of the search | Examiner |
|---|---|---|
| The Hague | 1 April 2016 | Trattner, Barbara |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ....................................................................
& : member of the same patent family, corresponding
   document

EPO FORM 1503 03.82 (P04C01)

## EP 3 017 762 A1

**ANNEX TO THE EUROPEAN SEARCH REPORT
ON EUROPEAN PATENT APPLICATION NO.**

EP 15 19 3735

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2016

| Patent document cited in search report | | Publication date | Patent family member(s) | | Publication date |
|---|---|---|---|---|---|
| US 4324259 | A | 13-04-1982 | DE | 2821223 A1 | 30-11-1978 |
| | | | FR | 2390937 A1 | 15-12-1978 |
| | | | GB | 1604251 A | 09-12-1981 |
| | | | US | 4324259 A | 13-04-1982 |
| WO 9919704 | A1 | 22-04-1999 | AU | 9297298 A | 03-05-1999 |
| | | | WO | 9919704 A1 | 22-04-1999 |
| US 5195536 | A | 23-03-1993 | NONE | | |
| US 4559953 | A | 24-12-1985 | CA | 1135525 A | 16-11-1982 |
| | | | DE | 3063057 D1 | 16-06-1983 |
| | | | DE | 8012386 U1 | 15-01-1981 |
| | | | EP | 0019321 A1 | 26-11-1980 |
| | | | GB | 2048485 A | 10-12-1980 |
| | | | JP | S564005 A | 16-01-1981 |
| | | | JP | S632042 B2 | 16-01-1988 |
| | | | US | 4559953 A | 24-12-1985 |

For more details about this annex : see Official Journal of the European Patent Office, No. 12/82

**EP 3 017 762 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61462811 A

- EP 12745140 A

**Non-patent literature cited in the description**

- **C.N. SMYTH.** The Guard-Ring Tocodynamometer. *Journal of Obstetrics and Gyneacology,* February 1957, vol. 64, 59-66